# EUROPEAN PATENT APPLICATION

(11) **EP 1 985 284 A1**
(43) Date of publication of application: **29.10.2008**
(21) Application number: 07008366.2
(22) Date of filing: 25.04.2007
(51) Int. Cl.: A61K 9/10

(54) **Vaccine formulation**

(71) Applicant: OctoPlus Sciences B.V., 2300 AS Leiden (NL)
(72) Inventor: The designation of the inventor has not yet been filed
(74) Representative: Kingma, Roelofke Leentje

(57) **Abstract**

The present invention describes a novel vaccine formulation comprising hydrogel-entrapped antigen. Such hydrogel-entrapped antigen may advantageously be used in the preparation of a vaccine formulation that may replace conventional multiple-administration vaccination schemes. Such vaccine formulation advantageously provides a prime and a booster dose of the vaccine with a single injection. The invention also relates to a method for immunizing a subject in need thereof, comprising administering to said subject said hydrogel-entrapped antigen or such vaccine formulation comprising hydrogel-entrapped antigen.

## Description

### FIELD OF THE INVENTION

The present invention relates to biodegradable hydnogel=entrapped antigen, as well as a vaccine formulation comprising free antigen and such polymer-entrapped antigen. The biodegradable polymers preferably comprise hydroxyethyl methacrylate-derivatized dextran. Moreover, the invention relates to such biodegradable hydrogel-entrapped antigen and/or vaccine formulation for use as a medicament, and the use of such biodegradable hydrogel-entrapped antigen and/or vaccine formulation in the preparation of a medicament for immunization of a subject. In further aspects, the invention relates to a kit for immunization of a subject comprising such lyophilized biodegradable hydrogel-entrapped antigen and/or vaccine formulation, and to a method of immunizing a subject in need thereof comprising administering to said subject such biodegradable hydrogel-entrapped antigen and/or vaccine formulation.

### BACKGROUND OF THE INVENTION

So far, a large number of vaccines effective in preventing various infectious diseases have been developed, and are continued to be developed. However, many of the currently used vaccines require multiple injections with the vaccine, which requirement imposes an economical burden as well as inconveniences to the subjects requiring vaccinations. In particular, it has been found that only about 30% of those subjects receiving the first injection return for the second injection. Statistically, therefore, only 9 out of 100 would complete an immunization process when three injections are prescribed, clearly demonstrating the need for a vaccine that can accomplish immunization by one single injection.

The development of a single-shot vaccine has mostly focused on using a microparticle, wherein a desired antigen is encapsulated with a biodegradable polymeric material, which releases the antigen slowly over a prescribed time period to accomplish the vaccination,

Among various biodegradable polymers, the hydrophobic polymers polyiactide(PLA), polyglycolide(PGA), and poly(lactide-co-glycolide)(PLGA) are generally known to be safe because they undergo in vivo hydrolysis to lactic acid and glycolic acid, which are physiologically acceptable monomers. The degradation rates of these polymers vary with the glycolide/lactide ratio and molecular weight thereof, and, therefore, the release of the drug can be sustained over several months by adjusting the molecular weight and glycolide/lactide ratio of the polymer as well as the particle size.

Since 1988, World Health Organization (WHO) has been sponsoring a number of studies to develop a single-shot vaccine for tetanus toxoid using the above-mentioned biodegradable polymers. However, thus far, no single-shot vaccine formulation has been put to practical use, mainly due to the deficiencies that the amount of antibody formed by the encapsulated formulation is only about 1/10 of that produced by a conventional alum formulation and that the result is not reproducible. This result has been ascribed to i) the use of an organic solvent for dissolving the biodegradable polymer, which organic solvent may reduce or eliminate the antigenicity of the antigen by denaturing it, ii) the formation of antigen aggregates having reduced antigenicity when the antigen contacts water, and iii) reduction or elimination of the antigenicity of the antigen due to interactions between the antigen and the hydrophobic biodegradable polymer.

Therefore, in order to prepare an efficient single-shot vaccine by encapsulating an antigen with a biodegradable polymer, one must use a biodegradable polymer which does not require the use of an organic solvent in the encapsulating step, or the antigen must be prevented from contacting an organic solvent during the process.

Thus, there have been many attempts to develop a process for preparing a single-shot vaccine which avoids undesirable interactions of an antigen with an organic solvent, a biodegradable polymer and water. Despite these efforts, a method capable of adequately protecting the antigenicity of an antigen during the process of encapsulating the antigen has not been found; and, accordingly, there has continued to exist a need to develop a single-shot vaccine formulation.

The hepatitis B virus model represents a good model of a system where a single-shot vaccination would be highly advantageous.

Infection with hepatitis B virus (HBV) represents a global public health problem and is the 10^{th} leading cause of death worldwide. HBV is classified as a member of the *Hepadnaviridae* family of viruses, which cause liver diseases in human and animals. HBV resides in the liver of infected humans, which it enters via the bloodstream. The virus replicates in hepatocytes and subsequently infects more liver cells.

HBV is transmitted by either skin puncture or mucous contact with infected body fluids. The highest concentration of HBV is found in the blood; however, the virus is present in and can be transmitted through all body fluids, such as saliva, semen and perspiration. HBV infection can also be aided through contaminated surfaces, such as needles and surgical tools, since the virus is resistant to breakdown and can survive and stay infectious outside the body for at least 7 days. The main routes of HBV transmission are perinatal transmission, transmission from child to child, unsafe injections and blood transfusions and sexual intercourse.

A person infected with HBV can carry the virus symptomatic (acute Hepatitis B) or asymptomatic. In both cases an infected person can either cure from the infection and develop lifelong immunity or become chronically infected, which, if untreated, lasts for life.

In people with acute HBV infection the incubation period and onset of symptoms ranges from 60 to 150 days. Symptoms of acute infection usually last for several weeks and include nausea, abdominal pain, jaundice and hepatomegaly. About 1-2% of the acute hepatitis cases are fatal, resulting from fulminant hepatitis. Chronic carriers of HBV may carry the virus for many years without any complications until symptoms of acute hepatitis arise or they are diagnosed with liver complications. Most chronic carriers die from liver disease, such as liver cirrhosis, which is a result of the immune system trying to get rid of the virus by repeatedly destroying hepatocytes, or by hepatocellular carcinoma (liver cancer), with 60-80% of the global cases estimated to result from HBV infection.

Infection with HBV is well preventable via vaccination. Currently commercially available vaccines against HBV infection include Engerix-B (GlaxoSmithKline), Recombivax-HB (Merck-Sanofi), Fendrix (GlaxoSmithKline), Genhevac (Sanofi-Aventis) and Sci-B-Vac (SciGen). The antigen used in all these preparations is the surface antigen of hepatitis B virus (HBsAg). The antigen may be expressed in and isolated from yeast cells (Engerix-B, Recombivax-HB, Fendrix), or from mammalian cells (Genhevac, Sci-B-Vac).

The current vaccination regimen against HBV consists of three injections; one prime injection and two booster injections that are routinely administered at 1 month and 4 to 6 months following the first (prime) injection. Vaccine efficacy after a full series of injections is estimated at 95%, with a duration of immunity of 15 years or more. However, compliance to this 3 doses regiment is relatively poor, especially in developing countries, mainly due to logistic reasons.

### SUMMARY OF THE INVENTION

Thus, in the art there is a need for vaccine formulations that improve overall compliance by providing more convenience to the subject to be immunized as well as to the medical personnel. Moreover, it would be advantageous if such vaccine formulation displays at least equal potency to the conventional multiple injection vaccines, improves the short-term protective immunity by inducing a faster onset of the appropriate immune response, and enhances the control of the direction of the induced specific immune response, e.g. biased towards a cellular immune response.

The biodegradable hydrogel-entrapped antigen and vaccine formulation according to the present invention aim to improve overall compliance by providing the convenience of a one shot vaccination.

In a first aspect, the present invention relates to a biodegradable hydrogel-entrapped antigen, wherein the biodegradable hydrogel comprises a network of polymer chains, which polymer chains are interconnected to one another through spacers, which spacers comprise a crosslinking unit and at least one bond which is hydrolysable under physiological conditions.

Also, the present invention relates to a single-shot vaccine formulation comprising free antigen, as well as such biodegradable hydrogel-entrapped antigen,

In a second aspect, the invention relates to such biodegradable hydrogel-entrapped antigen or vaccine formulation for use as a medicament.

In a further aspect, the invention is directed to the use of such biodegradable hydrogel-entrapped antigen or vaccine formulation in the preparation of a medicament for immunization of a subject.

The invention also relates to a kit for immunization of a subject, said kit comprising a lyophilized biodegradable hydrogel-entrapped antigen or vaccine formulation as defined in the present invention and reconstitution fluid suitable to dissolve and/or disperse the lyophilized biodegradable hydrogel-entrapped antigen or vaccine formulation, to a method for immunizing a subject in need thereof comprising administering to said subject a biodegradable hydrogel-entrapped antigen or vaccine formulation according to the invention, and to a pharmaceutical composition comprising a biodegradable hydrogel-entrapped antigen or vaccine formulation of the invention and one or more pharmaceutically acceptable carriers and/or excipients,

In a further aspect, the present invention relates to the use of adjuvant-free antigen as free antigen in the preparation of a single-shot vaccine formulation comprising free and biodegradable hydrogel-entrapped antigen for immunization of a subject.

### DETAILED DESCRIPTION OF THE INVENTION

The present invention relates to a biodegradable hydrogel-entrapped antigen, wherein the biodegradable hydrogel comprises a network of polymer chains, which polymer chains are interconnected to one another through spacers, which spacers comprise a crosslinking unit and at least one bond which is hydrolysable under physiological conditions.

The term 'antigen' is well known in the art and refers to a molecule that can induce an immune response. The antigen may be any type of antigen, and may be of any origin, such as bacterial or viral. Non-limiting examples of types of antigen are protein antigens, polysaccharide antigens, viral particles, live attenuated viruses and cells such as bacterial cells, inactivated whole viruses, and the like.

As herein used, the term 'hydrogel' refers to a water-swollen, three-dimensional network of crosslinked hydrophilic macromolecules. More specifically, the hydrogel used in the present invention consists of two interpenetrating networks interconnected to one another through hydrolysable spacers. Hydrogels generally contain at least 10% of the total weight (or volume) water.

The term "biodegradable" as used herein refers to its general meaning in the art. In accordance with the present invention, all types of biodegradable hydrogels can be used, provided that hydrolytically labile spacers cen be introduced in these structures. When introduced into the system of an animal or human body, the hydrogel structure is more or less gradually broken down. The degradation products do not need to be removed after introduction; they can simply be metabolized and/or excreted by the body.

Preferably, the hydrogels are based on water soluble polymer chains which contain at least a number of side groups having the capability to form links with other polymers through spacers, e.g. dextran or derivatized dextrans, while starches and starch derivatives, cellulose derivatives such as hydroxyethyl and hydroxylpropyl cellulose, polyvinylpyrrolidone, proteins, polyamino acids, polyvinyl alcohol, polyacrylates, polymethacrylates, polyethylene glycols that contain a number of monomer units capable of forming side chains, and so on, can also be used, as well as copolymers thereof.

The water-soluble polymer chains are interconnected to one another through spacers. The spacers comprise a crosslinking unit and at least one bond that is hydrolysable under physiological conditions. Non-limiting examples of crosslinking units are methacrylate units, acrylate units, vinyl ethers and vinyl esters, as well as other units known for this purpose by the person skilled in the art. Preferred crosslinking units according to the present invention are methacrylate and methacrylamide units, as it has been found that such crosslinking units are highly crosslinkable and yield hydrogels with a highly intricate network. The latter is particularly advantageous for controlled release purposes,

Generally, the hydrogel used in the present invention is made hydrolysable under physiological conditions by introducing at least one hydrolytically labile unit in the spacer located between two interconnected polymer chains. Using the hydrogel of the present invention, the release of biodegradable hydrogel-entrapped antigen can be controlled independently by adjusting the water content and/or the degree of crosslinking.

Preferably, the at least one bond that is hydrolysable under physiological conditions is selected from the group consisting of carbonate bonds, carboxylic acid ester bonds, urethane bonds, anhydride bonds, (hemi)acetal bonds, amide bonds, peptide bonds and mixtures of said bonds. In a preferred embodiment, the hydrogels used in the present invention comprise as the at least one bond that is hydrolysable under physiological conditions lactate, glycolate or ε-caprolactone ester bonds and/or carbonate bonds. A carbonate ester bond may e.g. be introduced by coupling carbonyldiimidazole activate HEMA to dextran, as is shown in European Patent EP 0 910 412 (the resulting product is hereinafter referred to as 'Dex-HEMA'), which is incorporated herein by reference. A lactate ester bond can be introduced in the hydrogel by introducing e.g. lactic acid monomer, glycolic acid monomer, lactic acid dimer, glycolic acid dimer, oligoglycolic acid, oligolactic acid, polyglycolic acid residues and/or polylactic acid residues or any combination of these between the polymer chain and the crosslinkable unit. A non-limiting example of such polymeric hydrogel is disclosed in European Patent EP 0 910 412 (Dex-lactate-HEMA). One skilled in the art will be capable of introducing such lactate, glycolate and/or ε-caprolactone ester bond and/or carbonate bond based on the disclosure of e.g. EP 0 910 412, which is herein incorporated by reference.

Preparation methods for hydrogels according to the present invention are e.g. disclosed in European patent EP 0 910 412. Hydrogels according to the present invention can suitably be prepared by first synthesizing spacers that contain at least one crosslinkable group and at least one hydrolytically labile group; coupling said spacers to a water-soluble polymer, and crosslinking the polymers obtained, preferably in the presence of the antigen to be entrapped within the hydrogel.

Preferably, the polymer chains are based on dextran or derivatised dextran. Dextran has the GRAS status, and is rernoved by the human or animal body without adverse effects.

In an embodiment, the biodegradable hydrogel comprises dextran derivatized with optionally hydroxylated alkyl methacrylate, preferably hydroxyethyl methacrylate-derivatized dextran. More preferably, the biodegradable hydrogel consists essentially of hydroxyethyl methacrylate-derivatized dextran (Dex-HEMA). In such hydroxyethyl methacrylate-derivatized dextran, a dextran backbone is substituted with HEMA side chains, The HEMA side chains are preferably coupled to the dextran backbone via a hydrolysable bond, preferably a carbonate bond. The preparation of Dex-HEMA is e.g. disclosed in EP 0 910 412. The Dex-HEMA may have any degree of substitution (number of substitutes per 100 glucopyranose residues), i.e. the dextran backbone may contain any number of HEMA substitutes per 100 glucopyranose residues. The controlled release characteristics may be tailored by varying the degree of subsitution. Dex-HEMA having a degree of substitution of between 3-30 may for example be used. Preferably, the biodegradable hydrogel comprises hydroxyethyl methacrylate-derivatized dextran having degree of substitution in the range of 3-15, yet more preferably in the range of 5-10, and most preferably in the range of 5-8. It was found in the present application that using such degree of substitution, suitable release profiles were obtained for HBsAg.

In one embodiment, the biodegradable hydrogel-entrapped HBsAg is entrapped in microparticles having the capability to form a hydrogel. Microparticle suspensions are easy to prepare and are easily used for injection. Microparticles can e.g. be prepared by dissolving Dex-HEMA (and optionally the antigen) in water, adding this solution to a PEG phase, and emulsifying the resultant yielding a water-in-water emulsion (with the Dex-HEMA phase (optionally containing the antigen) being dispersed within the PEG phase). After addition of a suitable initiator system, the methacrylate or methacrylamide groups polymerize yielding stable microparticles. Suitable initiator systems include a known initiator system of a tertiary amine and a persulphate, as disclosed in EP 0 910 412 and in EP 1 371 364, both of which are herein incorporated by reference.

In the context of the invention, microparticles should be understood as solid or semisolid particles having a diameter in the region of about 0.1 to about 500 µm, regardless of their shape or internal structure. For example, microparticles would also encompass microspheres and microcapsules. In a more preferred embodiment, the microparticles have a diameter from about 0.1 to about 300 µm. Selecting such particle size will also ensure that a suspension of these microparticles is well-syringeable and can be easily and conveniently administered via intramuscular or subcutaneous injection.

In a preferred embodiment, the antigen is HBsAg antigen.

The term 'HBsAg' is well known in the art and refers to the Hepatitis B surface antigen. It is a part of the hepatitis B virus that, when in the blood, is one of the earliest markers of infection with hepatitis B virus, appearing even before symptoms, It is a complex macromolecular aggregate composed of protein, carbohydrates (in the form of glycoproteins) and lipids. Recombinant HBsAg can be produced with yeast cells (e.g. from *Hansenula polymorpha*) or mammalian cells (e.g. Chinese hamster ovary [CHO] cells). Depending on its source, the HBsAg particle may differ somewhat. For example, CHO-HBsAg contains 3 proteins, S, preS1, and preS2, each in two forms of glycosylation, whereas the Hansenula-HBsAg contains only the non-glycosylated S peptide. As such, the term 'HBsAg' as used herein encompasses many variations of the HBsAg particle, including, but not limited to, HBsAg particles comprising as only protein S antigen (as mostly obtained from yeast), HBAg particles comprising S antigen, and proS1 protein, HBsAg particles comprising S antigen, preS1 protein, and preS2 protein, optionally glycosylated, and the like. Generally, the term 'HBsAg' as used throughout the present application refers to a HBsAg particle comprising at least the S antigen, glycosylated or non-glycosylated.

In a further preferred embodiment, the HBsAg antigen is HBsAg antigen derived from Chinese Hamster Ovarian (CHO) cells. It was found that such CHO-derived HBsAg was considerably more immunopotent than yeast-derived HBsAg.

In a further aspect, the present invention is directed to a single-shot vaccine formulation comprising free antigen, and biodegradable hydrogel-entrapped antigen as defined above, In such single-shot vaccine formulation the free antigen serves as a prime dose, whereas the biodegradable hydrogel-entrapped antigen serves as boost dose.

The term 'five antigen' as used herein refers to antigen that is not entrapped within a polymer (micro)particle or network. It may be soluble, dispersible, or it may be complexed with an adjuvant such as aluminium or non-adjuvanted, as long as it is not entrapped in a polymer particle or network. The free antigen is advantageously present in an amount sufficient to ensure priming of the immune response for vaccination. The term 'aluminium' or 'alum' as used herein refers to any aluminium derivatives, e.g. aluminium salts and aluminium hydroxide.

In a preferred embodiment, the free antigen is free of aluminium adjuvant, i.e. is not complexed with aluminium, Surprisingly, it was found for HBsAg that using free antigen that was not complexed with aluminium immunization was more potent than using free antigen that was complexed with the adjuvant aluminium. This is all the more surprising since for immunization with HBsAg complexation with aluminium is required in the current vaccination regime to generate a sufficient immune response.

Furthermore, the present invention relates to a biodegradable hydrogel-entrapped antigen as herein defined or a vaccine formulation as herein defined for use as a medicament. In an embodiment wherein the antigen is HBsAg, the medicament preferably serves to immunize a subject against Hepatitis B.

Also, it relates to the use of a biodegradable hydrogel-entrapped antigen as herein defined or a vaccine formulation as herein defined in the preparation of a medicament for immunization of a subject, Such medicament may be in any physical form known to the skilled person, such as liquid form or frozen form. It may also be lyophilized, spraydried or solidified in any other way.

In a further aspect, the present invention is directed to a kit for immunization of a subject, said kit comprising a lyophilized biodegradable hydrogel-entrapped antigen, such as HBsAg, as herein defined or vaccine formulation as herein defined and reconstitution fluid suitable to dissolve and/or disperse the lyophilized biodegradable hydrogel-entrapped antigen or vaccine formulation. The reconstitution fluid may be any fluid capable of dissolving and/or dispersing the lyophilized biodegradable hydrogel-entrapped antigen or vaccine formulation. Non-limiting examples of suitable reconstitution fluids include physiological salt solution, water for injection, and isotonic sucrose solution.

In yet a further aspect, the present invention relates to a method for immunizing a subject in need thereof comprising administering to said subject a biodegradable hydrogel-entrapped antigen as herein defined such as HBsAg or a vaccine formulation as defined herein. Subjects in need for immunization against Hepatitis B include without limitation children, travellers to endemic areas such as South East Asia, and inhabitants of e.g. Asia and South America. Administration may be via any route, but preferably takes place by means of injection, preferably intramuscular or subcutanous injection, more preferably by subcutanous injection.

The present invention also relates to a pharmaceutical composition comprising a biodegradable hydrogel-entrapped antigen as herein defined or a vaccine formulation as defined herein, and one or more pharmaceutically acceptable carriers and/or excipients.

Suitable carriers and/or excipients are well known to the person skilled in the art and can e.g. be found in Remington's Pharmaceutical Sciences.

The pharmaceutical composition of the invention may be designed, formulated and processed so as to be suitable for parenteral administration. As used herein, parenteral administration includes any invasive route of administration, such as subdermal, intradermal, subcutaneous, intramuscular, locoregional, intratumoral, intraperitoneal, interstitial, intralesional, with some less preference in the context of this invention also intravenous, intraarterial etc. Highly preferred routes of administration of the composition are subcutaneous and intramuscular injection or implantation.

Being suitable for parenteral administration means in particular that the pharmaceutical composition preferably is sterile and complies with the requirements of the current pharmacopoeias with regard to the content of endotoxins, osmolality, etc. The excipients are preferably selected to be safe and tolerable for parenteral administration. In a further aspect, the composition is formulated to be relatively isotonic (or isoosmotic), such as in the region of about 150 to 500 mOsmol/kg, and preferably in the region of about 250 to 400 mOsmol/kg. Furthermore, the pH should be approximately in the physiological range in order to avoid pain and local intolerance upon injection. Preferably, the pH of the final composition is in the region of about 4 to 8.5, and more preferably in the region of about 5.0 to 7.5.

The invention also relates to the use of adjuvant-free antigen as free antigen in the preparation of a single-shot vaccine formulation comprising free and biodegradable hydrogel-entrapped antigen for immunization of a subject. It has been found for the first time that elimination of adjuvant, in the present case aluminium, in the free antigen part of the vaccine formulation according to the present invention (in the present case HBsAg), gave significantly better results than incorporation of adjuvant, in the present case aluminium. Evidently, the difference between adjuvanted and non-adjuvanted (adjuvant-free) antigen as free antigen in the preparation of a single-shot vaccine formulation comprising free and biodegradable hydrogel-entrapped antigen will be envisaged only for antigens that are conventionally administered in complex with adjuvant.

The invention will hereinafter be explained in more detail with reference to the examples and the figures, which are in no way to be construed as limiting the scope of the present invention. The terms 'OctoVAX' and 'OctoVAX microspheres' as used hereinafter refer to microparticles composed of Dex-HEMA, optionally comprising HBsAg antigen derived from either yeast or CHO cells.

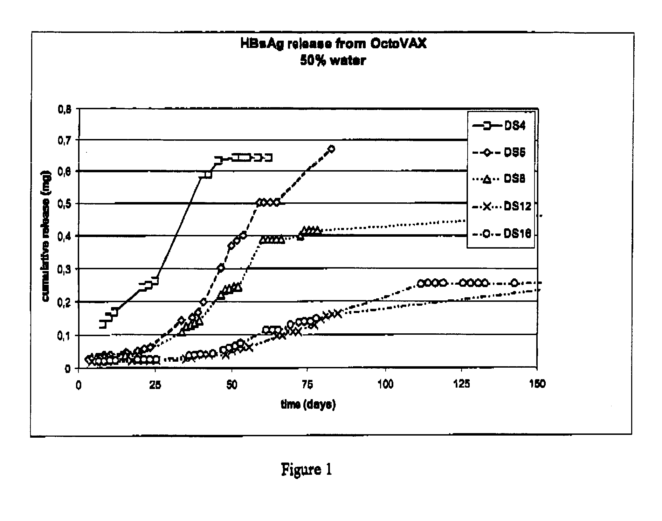

Figure 1 shows the *in vitro* yeast-HBsAg release at pH 8.4 from OctoVAX microspheres having a water content of 50% and a HBsAg load of 0.61 mg. The OctoVAX microspheres were prepared from Dex-HEMA having various degrees of substitution (DS4, DSS, DS8, DS12 and DS16, respectively). The measurements were performed in duplicate for OctoVAX microspheres having DS5, DS8, DS12 and DS16, and a single experiment was performed for OctoVAX microspheres having DS4. The time axis is based on conversion of the pH8.4 accelerated release data to real-time release data by factors obtained in direct comparison of accelerated vs. real-time release.

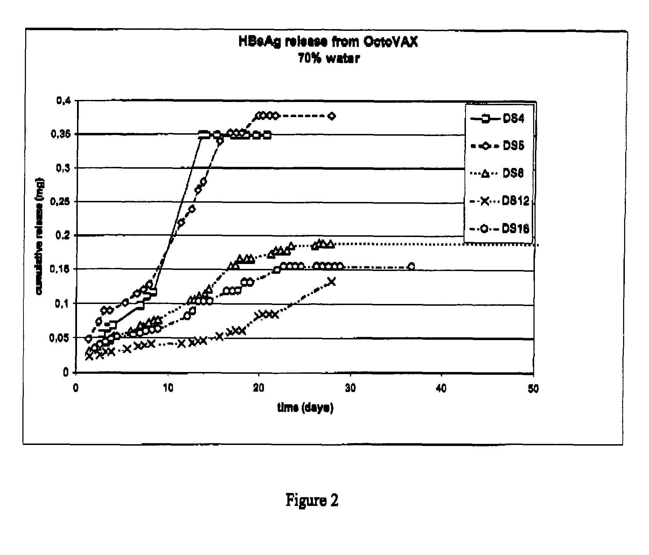

Figure 2 shows the *in vitro* yeast-HHsAg release at pH 8.4 from OctoVAX microspheres having a water content of 70% and a HBsAg load of 0.366 mg. The OctoVAX microspheres are prepared from Dex-HEMA having various degrees of substitution (DS4, DS5, DS8, DS12 and DS16, respectively). The measurements were performed in duplicate for OctoVAX microspheres having DS5, DS8, DS12 and DS16, and a single experiment was performed for OctoVAX microspheres having DS4. The time axis is based on conversion of the pH8.4 accelerated release data to real-time release data by factors obtained in direct comparison of accelerated vs. real-time release.

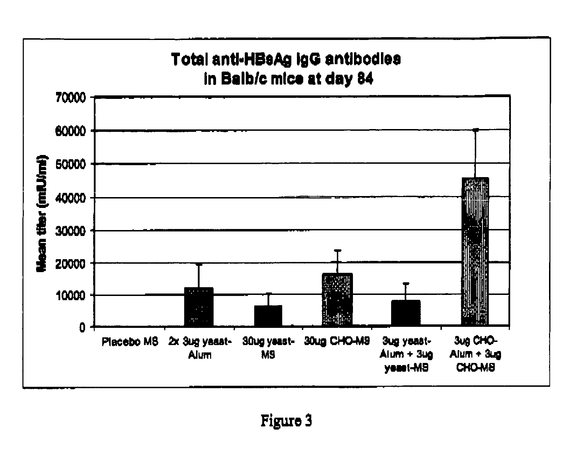

Figure 3 shows mean titres as induced in Balb/c mice by a selection of vaccinations. Vaccinations were carried out using 2 types of HBsAg: yeast-derived HBsAg and CHO-derived HBsAg. Control animals injected with placebo microspheres at day 0 or with alum-adjuvanted yeast-derived HBsAg (yeast-Alum) on day 0 and day 28 were compared with selected formulations containing either yeast HBsAg or CHO HBsAg. For each vaccination regime, the bar indicates the mean titre on day 84. The mean of all animals per group was determined and the error bars indicate the SEM.

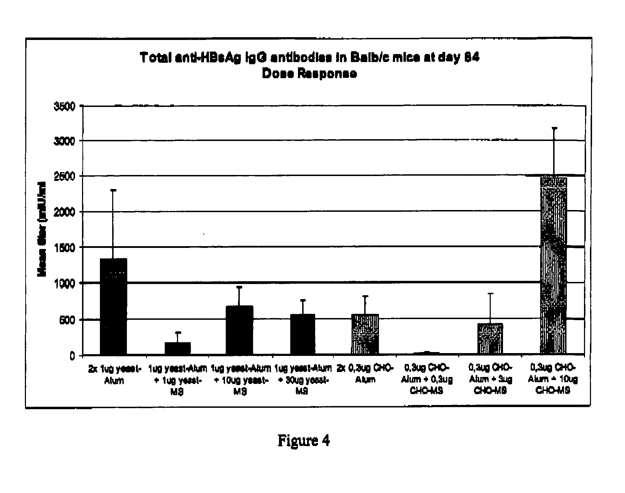

Figure 4 demonstrates the immunopotency of increasing dose OctoVAX microsphere formulations containing either yeast-derived HBsAg or CHO-derived HBsAg. Mean titre values for each group are indicated for day 84. The error bars indicate the SEM.

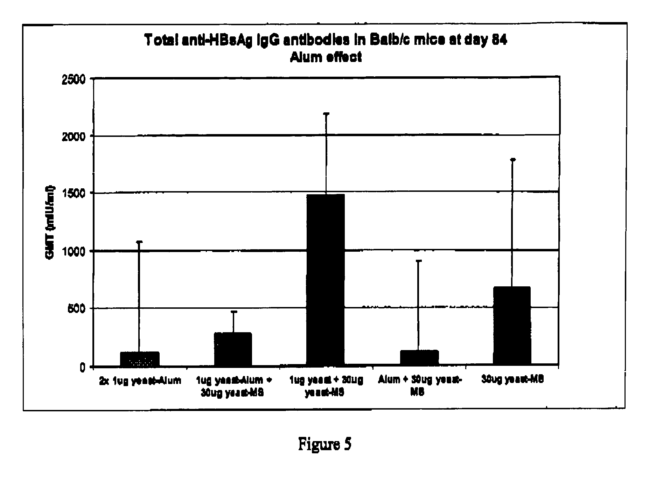

Figure 5 demonstrates the immunopotency of different prime compositions in combination with a consistent OctoVAX microsphere-dose containing yeast-derived HBsAg. Geometric mean titre values are indicated for each group for day 84. The error bars indicate the SEM.

### EXAMPLES

### MATERIALS AND METHODS

### Chemicals

Yeast-derived HBsAg was obtained from the Serum Institute of India Ltd., India, CHO-derived HBsAg was obtained from SciGen IL Ltd., Israel.

Dex-HEMA DS4 was obtained from Utrecht University, Utrecht, The Netherlands. Dex-HEMA DS5, DS8, DS12, DS16, DS20 and DS30 were obtained from PSCG, Groningen, The Netherlands. Dextran 40.000 PH EUR was purchased from Pharmacia, Uppsala, Sweden. PEG 10.000 was obtained from Chempri, Raamsdonkveer, The Netherlands.

Sodium hydroxide (NaOH), boric acid (H₃BO₃), sodium dihydroxyphosphate monohydrate (NaH₂PO₄.H₂O), and disodium hydroxyphosphate dihydrate (Na₂HPO₄,2H₂O) were obtained from Merck, Darmstadt, Germany. Phosphate buffered saline (PBS, pH 7.4) was purchased from Invitrogen (Gibco), Paisly, Scotland, UK. Methanol (MeOH) HPLC grade was obtained from Biosolve, Valkenswaard, The Netherlands. Tween 20 was obtained from Merck, Hohenbrunn, Germany. N,N,N',N'-Tetramethylethylenediamine (TEMED), Sodium peroxodisulphate (NaPS), Glycine and sodium borate (Na₂B₄O₇) were purchased from Sigma-Aldrich, Steinheim, Germany. HCl was obtained from Sigma, Seelze, Germany. All other chemicals were of chemical grade purity.

### Procedure for the preparation of a 5 g batch of OctoVAX microspheres

In a 15 ml greiner tube, Dex-HEMA was dissolved in HBsAg solution and additional buffer (for placebo batches, Dex-HEMA was dissolved in buffer only). PEG solution and TEMED solution were added and the mixture was emulsified using a vortex for 1 minute at maximum speed. KPS solution was added, the tube was gently tumbled three times and then left to polymerize for 1 hour at RT.

The microspheres were washed 5-fold by centrifuging the tubes at 3500 rpm (1^{st} time 20 minutes, subsequent times 7 minutes). After each centrifugation cycle the supernatant was removed and replaced by 5.0 ml fresh buffer. The microspheres were resuspended. After the last centrifugation cycle the supernatant was replaced by 5.0 ml of the release buffer and the microspheres were resuspended.

### Analytical setup

### Automated IVR

For the automated IVR a Perkin Elmer HPLC system was used. The HPSEC column used was a TSKgel G5000 PWXI column (300x7.8 mm) with a TSKgel PWH Guadcolumn (75x7.5 mm) installed, The column temperature was set to 24°C and the peltier tray was set to 37°C. The needle was flushed with MeOH/H2O (50:50). The flow used was 1.0 ml/min and the runtime was 22 minutes. The injection volume was set to 20 µl. With every sample an additional 10 µl was taken up for preflushing the needle. UV detection was performed at 210 nm using a UV detector. A mobile phase of PBS (pH 7.0) with 1% Tween 20 was used (isocratic).

Calibration curves of the two HBsAg antigens were obtained for each separate sequence.

For automated IVR two samples of 1.5 ml were taken per batch from the homogenized suspensions and transferred to HPLC vials. The vials were centrifuged for 10 minutes at 3500 rpm and then placed into the autosampler tray, which was set to 37°C. Samples were taken from the supernatant at predefined time points and were directly injected onto the column.

### Microscopy and particle size analysis

An Olympus microscope with 10x, 40x and 100x objectives installed on normal light modus was used to obtain morphology images. For particle size analysis a Malvern Mastersizer was used. For microscopy 1 droplet of each homogenized batch was used. For particle size analysis as many homogenized sample as needed to obtain enough obscuratian were used.

### Nitrogen analysis

A Thermo Electron elemental nitrogen analyzer was used for determination of encapsulation efficiency and remaining content of HBsAg in the samples. An injection volume of 500 µl was used. A calibration curve of glycine was used to calculate the amount of nitrogen in the samples.

For N-analysis for determination of encapsulation efficiency, 1 ml of each homogenized batch was diluted with 3 ml WFI. A stirrer bar was placed in the vials to ensure homogenization during analysis. The same was done for the placebo batches.

For N-analysis for determination of remaining antigen after IVR, 0.5 ml of the homogenized sample was transferred into a 1.5 ml Eppendorf cup and centrifuged for 30 min. at 13000 rpm. The supernatant was carefully replaced with 0.5 ml WFI and the microspheres were resuspended. This was repeated. The sample was transferred into a vial that already contained 1 ml WFI, 0.5 ml WFI was used to rinse the Eppendorf cup and added to the vial. A stirrer bar was added. Total dilution: 4x.

### Example 1 In vitro release from OctoVAX microspheres having various degrees of substitution

OctoVAX microspheres were prepared using Dex-HEMA having various degrees of substitution (DS; DS4-16) (hereinafter e.g. referred to as "OctoVAX DS 4-16"). The microspheres were loaded with either 0.61 mg or 0.366 mg yeast-derived HBsAg, and were prepared in such a way that the water content was either 50% or 70%. Subsequently, the release profiles at pH 8.4 of HBsAg from such microspheres were determined.

For microspheres having a water content of 50% and a HBsAg load of 0.61 mg, it was found that the microspheres obtained with Dex-HEMA having a DS of 4 or 5 were not completely round in shape, whereas those obtained with Dex-HEMA having a DS of 8, 12 or 16 were perfectly round in shape.

The release profiles obtained from the microspheres are shown in Figure 1. The profiles of OctoVAX DS 12 or DS 16 were found to be similar and showed a lag time of approximately 4 weeks, followed by a gradual release during 10-12 weeks. OctoVAX DS5 and DS8 showed a lag time of about 3 weeks and released their contents in approximately 7 weeks. With OctoVAX DS4 no lag time was determined and release was faster than for OctoVAX DS5.

For microspheres having a water content of 70% and an HBsAg load of 0.366 mg, it was found that the microspheres obtained with OctoVAX DS 4 were not completely round in shape. From OctoVAX DS5 onwards, spherical particles were found.

The release profiles obtained from the microspheres are shown in Figure 2. The profiles of OctoVAX DS4 and OctoVAX DS5 were similar; a more or less linear profile was obtained. Both types of microspheres released their complete contents within approximately 15 days. OctoVAX DS8 microspheres released less HBsAg and the release rate was slower than for OctoVAX DS4 and DS5. OctoVAX DS16 microspheres released their content only slightly slower than OctoVAX DS8 microspheres.

### Example 2 Animal studies using single-shot vaccine formulation

In these studies, the antigen source was either Chinese hamster ovarian cells (CHO), or HBsAg produced in yeast. In contrast to the yeast material that consist of a single non-glycosylated protein, the CHO material consists of HBsAg particles made up of 3 proteins that are glycosylated. The CHO material as such mimics the HBsAg particles that are naturally occurring in blood of HBV infected humans. The release profiles for the yeast material formulations (DS5, 50% water) and CHO material formulations (DS8, 70%) show a pulsed release. After immunisation on day 0, serum was collected with 28-day intervals until day 84. Additionally, in the study presented in Table 1 serum was also collected at day 170 to obtain an indication about effects on the duration of the response. Antibody titers were determined with the commercially available BioRad Monolisa HBs3.0 kit, and presented as mIU/ml in reference to the international standards included in this kit. In Table 1 the number of animals with serum antibody levels above those regarded as seroprotection limits are indicated.

**Table 1 Seroprotection in mice immunized subcutaneously**

| **Treatment** | **Day of immunisation** | **d28** | **d56** | **d84** |
|---|---|---|---|---|
| Buffer | 0 and 28 | 0/10^{a} | 0/10^{a} | 0/10^{a} |
| Placebo MS | 0 | 0/10 | 0/10 | 0/10 |
| 2x 3 µg yeast-Alum | 0 and 28 | 3/10 | 9/10 | 10/10 |
| 1x 3 µg yeast-Alum | 0 | 4/10 | 10/10 | 10/10 |
| 3 µg yeast-Alum + Placebo MS | 0 | 5/10 | 10/10 | 10/10 |
| 3 µg yeast-MS | 0 | 0/9 | 0/9 | 0/8 |
| 3 µg yeast-Aluin + 3 µg yeast-MS | 0 | 3/10 | 10/10 | 9/9 |
| 30 µg yeast-MS | 0 | 6/10 | 8/9 | 8/9 |
| 3 µg yeast-Alum + 30 µg yeast-MS | 0 | 8/10 | 10/10 | 10/10 |
| 3 µg CHO-MS | 0 | 0/10 | 2/10 | 3/10 |
| 3 µg CHO-Alum + 3 µg CHO-MS | 0 | 10/10 | 10/10 | 9/9 |
| 30 µg CHO-MS | 0 | 8/10 | 9/10 | 9/10 |

| | | | | |
|---|---|---|---|---|
| ^{a} number of mice with antibody titers higher than 30 mIU/ml as compared to the total number of mice in the group | | | | |

It is generally accepted that an antibody titer of>10 mIU/ml represents seroprotection in humans. The animal immunization data indicate that seroconversion rates with the HBsAg vaccines combining an alum-adjuvanted HBsAg prime and a boost using OctoVAX-entrapped HBsAg (single-shot vaccination) are comparable to those achieved by conventional immunization. Notably, the number of seroprotected animals at day 28 tends to be higher for the single-shot vaccination than for the conventional immunization, especially with high doses of microspheres.
In addition to the seroconversion/seroprotection rates, the level of antibody induction is indicative of the potency of a vaccine. In Figure 3, the antibody titers for a select group of treatments is presented as mean titers. The general impression of these studies is that relatively high titers can be reached with HBV-OctoVAX formulations, albeit with relatively high dose of microspheres (Figure 3). Furthermore, antibody titres at day 170 indicate that the duration of the response induced by microsphere containing vaccines is not significantly different from the response induced by conventional 2 shot aluminium-HBsAg vaccination.
Figure 3 shows that the CHO-derived HBsAg is intrinsically more potent in inducing antibodies as can be seen by comparing the 30 µg MS-CHO with the 30 µg MS-yeast, as well as by comparing 3 µg prime CHO + 3 µg MS-CHO with 3 µg prime yeast + 3 µg MS-yeast. The titers induced by the prime-boost combination with 3 µg each, are approximately 10 fold higher with the CHO HBsAg compared to yeast material.

### Example 3 Determination of discriminating dose of HBV-OctoVAX

Based on the previous animal studies, a second animal study was performed to determine the dose of single shot (prime-boost) HBsAg-OctoVAX formulation at which discrimination between variations in composition can be determined. The free alum-HBsAg dose was reduced to 1 µg for yeast-derived HBsAg and to 0,3 µg for CHO-derived HBsAg. Increasing doses of OctoVAX microspheres containing HBsAg were admixed with a constant amount of free alum-HBsAg. In addition, the importance of the composition of the prime dose was studied by comparing alum-HBsAg prime with non-adjuvanted prime.
Serum was collected at at 28-day intervals until day 84. Antibody titers were determined with a commercially available EIA kit (BioRad Monolisa HBs3.0), and presented as mIU/ml in reference to the international standards included in this kit.

In Table 2 below, the seroconversion rates are indicated. A clear dose effect of the microspheres can be observed. There appears to be a threshold dose above which the microspheres increase the efficacy of the immunization above that of the prime dose. For both yeast and CHO HBsAg formulations this threshold was found to be 10 µg. Notably, the number of seroprotected animals at day 28 tended to be higher for the single-shot vaccination using free HBsAg in combination with OctoVAX-entrapped HBsAg than for the conventional immunization, especially with high doses of OctoVAX microspheres. Additionally, immunization with a prime-boost combination containing non-adjuvanted HBsAg as the prime component seems to be more effective in inducing an early response. On day 28, already 80% of the animals had developed detectable antibody titers.

The dose effect that was already reflected by the seroprotection numbers in Table 4, was also observed when the mean titres of the different doses were compared. Both for yeast-derived HBsAg and for CHO-derived HBsAg (Figure 4), a clear dose effect was observed. Again, there clearly was a threshold dose above which the response was elevated compared to the prime dose only:

**Table 2. Seroconversion in mice immunised subcutaneously**

| **Treatment** | **Day of immunisation** | **d28^{a}** | **d56^{a}** | **d84^{b}** |
|---|---|---|---|---|
| 1 µg yeast-Alum | 0 | 0/9 | 5/9 | 4/9 |
| 1 µg yeast-Alum + 1 µg yeast-MS | 0 | 1/10 | 1/10 | 5/10 |
| 1 µg yeast-Alum + 3 µg yeast-MS | 0 | 1/10 | 3/10 | 6/10 |
| 1 µg yeast-Alum + 10 µg yeast-MS | 0 | 1/10 | 8/10 | 10/10 |
| 1 µg yeast-Alum + 30 µg yeast-MS | 0 | 4/9 | 8/9 | 7/8 |
| 0,3 µg CHO-Alum | 0 | 0/10 | 1/10 | 2/10 |
| 0,3 µg CHO-Alum + 0,3 µg CHO-MS | 0 | 2/10 | 2/10 | 3/9 |
| 0,3 µg CHO-Alum + 1µg CHO-MS | 0 | 0/10 | 1/10 | 2/10 |
| 0,3 µg CHO-Alum + 3 µg CHO-MS | 0 | 1/10 | 1/10 | 2/10 |
| 0,3 µg CHO-Alum + 10 µg CHO-MS | 0 | 6/10 | 8/10 | 9/10 |
| 1 µg yeast + 30 µg yeast-MS | 0 | 8/10 | 9/10 | 9/9 |
| MS placebo | 0 | 0/10 | 0/10 | 0/10 |
| 30 µg yeast-MS | 0 | 5/10 | 8/10 | 8/10 |
| Alum + 30 µg yeast-MS | 0 | 1/10 | 4/9 | 7/9 |
| 2x 1 µg yeast-Alum | 0 and 28 | 1/10 | 6/10 | 7/10 |
| 2x 0,3 µg CHO-Alum | 0 and 28 | 1/10 | 6/10 | 8/10 |

| | | | | |
|---|---|---|---|---|
| ^{a} number of mice with antibody titers higher than 30 mIU/ml as compared to the total number of mice in the group. ^{b} number of mice with antibody titers higher than 6 mIU/ml as compared to the total number of mice in the group | | | | |

The immunopotency of a vaccine combining the microspheres as a booster component with non-adjuvanted HBsAg as a prime component was surprisingly high (Figure 5). Geometric Mean Titre (GMT) of the different doses were compared. The GMT is less sensitive to extremely high or low titers than the mean titre. Comparing alum-yeast 1 µg + MS30 with yeast 1 µg + MS30 (which contain equal total amounts of HBsAg), it was observed that for single-shot vaccination using free HBsAg without alum the GMT was 3-10 fold higher (Student's t-test P value< 0,02 for day 28 and 84).

## Claims

1. Biodegradable hydrogel-entrapped antigen, wherein the biodegradable hydrogel comprises a network of polymer chains, which polymer chains are interconnected to one another through spacers, which spacers comprise a crosslinking unit and at least one bond which is hydrolysable under physiological conditions.

2. The biodegradable hydrogel-entrapped antigen according to claim 1, wherein the at least one bond that is hydrolysable under physiological conditions is selected from the group consisting of carbonate bonds, carboxylic acid ester bonds, urethane bonds, anhydride bonds, (hemi)acetal bonds, amide bonds, peptide bonds and mixtures of said bonds.

3. The biodegradable hydrogel-entrapped antigen according to any of claims 1 or 2, wherein the polymer chains are based on dextran or derivatised dextran.

4. The biodegradable hydrogel-entrapped antigen according to any of the preceding claims, wherein the biodegradable hydrogel comprises hydroxyethyl methacrylate-derivatized dextran.

5. The biodegradable hydrogel-entrapped antigen according to claim 4, wherein the biodegradable hydrogel consists essentially of hydroxyethyl methacrylate-derivatized dextran.

6. The biodegradable hydrogel-entrapped antigen according to any of claims 4 or 5, wherein the biodegradable hydrogel comprises hydroxyethyl methacrylate-derivatized dextran having degree of substitution in the range of 3-15.

7. The biodegradable hydrogel-entrapped antigen according to claim 6, wherein the biodegradable hydrogel comprises hydroxyethyl methacrylate-derivatized dextran having degree of substitution in the range of 5-10.

8. The biodegradable hydrogel-entrapped antigen according to claim 7, wherein the biodegradable polymers comprise hydroxyethyl methacrylate-derivatized dextran having degree of substitution in the range of 5-8.

9. The biodegradable hydrogel-entrapped antigen according to any of the preceding claims, wherein the biodegradable hydrogel-entrapped antigen is entrapped in microparticles having the capability to form a hydrogel.

10. The biodegradable hydrogel-entrapped antigen according to any of the preceding claims, wherein the antigen is a HBsAg antigen.

11. The biodegradable hydrogel-entrapped antigen according to claim 10, wherein the HBsAg antigen is HBsAg antigen derived from Chinese Hamster Ovarian (CHO) cells.

12. A vaccine formulation comprising free antigen and biodegradable hydrogel-entrapped antigen according to any of claims 1-11.

13. A vaccine formulation according to claim 12, wherein the free antigen is free of adjuvant.

14. A vaccine formulation according to claim 13, wherein the free antigen is free of aluminium adjuvant.

15. Biodegradable hydrogel-entrapped antigen according to any of claims 1-11 for use as a medicament.

16. Vaccine formulation according to any of claims 12-14 for use as a medicament.

17. Use of a biodegradable hydrogel-entrapped antigen according to any of claims 1-11 in the preparation of a medicament for immunization of a subject.

18. Use of a vaccine formulation according to any of claims 12-14 in the preparation of a medicament for immunization of a subject.

19. Kit for immunization of a subject, said kit comprising a lyophilized biodegradable hydrogel-entrapped antigen according to any of claims 1-11 and reconstitution fluid suitable to dissolve and/or disperse the lyophilized biodegradable hydrogel-entrapped antigen.

20. Kit for immunization of a subject, said kit comprising a vaccine formulation according to any of claims 12-14 and reconstitution fluid suitable to dissolve and/or disperse the lyophilized vaccine formulation.

21. Method for immunizing a subject in need thereof comprising administering to said subject a biodegradable hydrogel-entrapped antigen according to any of claims 1-11.

22. Method for immunizing a subject in need thereof comprising administering to said subject a vaccine formulation according to any of claims 12-14,

23. Pharmaceutical composition comprising biodegradable hydrogel-entrapped antigen as defined in any of claims 1-11 and one or more pharmaceutically acceptable carriers and/or excipients.

24. Pharmaceutical composition comprising a vaccine formulation as defined in any of claims 12-14 and one or more pharmaceutically acceptable carriers and/or excipients.

25. Use of adjuvant-free antigen as free antigen in the preparation of a single-shot vaccine formulation comprising free and biodegradable hydrogel-entrapped antigen for immunization of a subject.
